(19) **Europäisches Patentamt / European Patent Office / Office européen des brevets**

(11) **EP 4 248 977 A1**

(12) # EUROPEAN PATENT APPLICATION
published in accordance with Art. 153(4) EPC

(43) Date of publication:
**27.09.2023 Bulletin 2023/39**

(21) Application number: **21894716.6**

(22) Date of filing: **18.11.2021**

(51) International Patent Classification (IPC):
*A61K 31/675* (2006.01)     *A61K 9/08* (2006.01)
*A61K 47/10* (2017.01)     *A61K 47/26* (2006.01)
*A61K 47/44* (2017.01)     *A61P 9/00* (2006.01)

(52) Cooperative Patent Classification (CPC):
**A61K 9/08; A61K 31/675; A61K 47/10;
A61K 47/26; A61K 47/44; A61P 9/00**

(86) International application number:
**PCT/JP2021/042404**

(87) International publication number:
**WO 2022/107843 (27.05.2022 Gazette 2022/21)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **19.11.2020  JP 2020192606**

(71) Applicant: **Nippon Kayaku Kabushiki Kaisha
Tokyo 100-0005 (JP)**

(72) Inventors:
• **ONDA Takeshi**
  **Tokyo 115-8588 (JP)**
• **KITAGAWA Masayuki**
  **Tokyo 115-8588 (JP)**
• **IGO Naoko**
  **Tokyo 115-8588 (JP)**
• **HARA Kazuhisa**
  **Tokyo 115-8588 (JP)**

(74) Representative: **Godemeyer Blum Lenze
Patentanwälte
Partnerschaft mbB - werkpatent
An den Gärten 7
51491 Overath (DE)**

(54) **COMPOSITION CONTAINING WATER-SOLUBLE DRUG CAPABLE OF INDUCING VASCULAR DISORDER, SOLUTION FOR USE IN PREPARATION OF ADMINISTRATION SOLUTION CONTAINING WATER-SOLUBLE DRUG CAPABLE OF INDUCING VASCULAR DISORDER, KIT, VASCULAR DISORDER PREVENTING AGENT, AND SOLUTION CONTAINING NONIONIC SURFACTANT**

(57)     Alleviation of vascular disorder such as vasculitis or angialgia is required in the use of a water-soluble drug causing vascular disorder. According to the present invention, a composition containing a water-soluble drug causing vascular disorder; a solution for use in preparation of an administration solution containing a water-soluble drug causing vascular disorder; and a kit including a water-soluble drug preparation causing vascular disorder and the aforementioned solution, each of which contains a nonionic surfactant in an amount necessary for alleviation of vascular disorder, are provided.

[Fig. 1]

**Description**

Technical Field

The present invention relates to a composition containing a water-soluble drug causing vascular disorder, a solution for use in preparation of an administration solution containing a water-soluble drug causing vascular disorder, a kit, an agent for suppressing vascular disorder, and a solution containing a nonionic surfactant.

Background Art

It has been reported that a plurality of pharmaceutical preparations for use in intravenous administration cause vascular disorder such as vasculitis or angialgia. For example, it has been known that a fosaprepitant preparation that is a pharmaceutical preparation for use in intravenous injection causes vascular disorder such as angialgia or vasculitis. Non-Patent Document 1 discloses that the onset frequency of angialgia (vasculitis) is 20% or more regarding the fosaprepitant preparation in clinical tests, and Non-Patent Document 2 discloses that the measures for alleviating the onset of vascular disorder have been studied in clinical sites.

It is not difficult to imagine that the onset of vascular disorder such as vasculitis or angialgia may discourage patients from treatments. Non-Patent Document 3 discloses the onset risk of vascular disorder in individual anticancer agents and the importance of safety management. In addition, Non-Patent Document 3 discloses that the factors for the onset of vasculitis or angialgia include the pH of a preparation, the cytotoxicity of a drug itself, and the like, and to address this problem, the inflammation can be suppressed by the combined use of dexamethasone having anti-inflammatory action and the like. However, as described in Non-Patent Document 4, dexamethasone has various side effects and it is difficult to manage its use. Accordingly, it has been desired to develop a method of preventing angialgia and/or vasculitis, which has a few side effects.

Prior Art Documents

Non-Patent Documents

Non-Patent Document 1: Fosaprepitant (product name: PROEMEND) Package insert
Non-Patent Document 2: "Measures for Alleviating Phlebitis Caused by Fosaprepitant Injection," Japanese Journal of Cancer and Chemotherapy, volume 42, issue 3, pp. 323-326, 2015 (Cancer and Chemotherapy, K.K.)
Non-Patent Document 3: "Book for Fully Understanding about Cancer Therapeutic Drugs," 1st edition, (Shorinsha Inc.)
Non-Patent Document 4: Journal of Japanese Society of Pharmaceutical Oncology, Vol. 14 (April, 2020)
Non-Patent Document 5: Dexamethasone Sodium Phosphate Product Attachments

Summary of Invention

Object to be Solved by the Invention

It is an object of the present invention to alleviate vascular disorder attended with administration of a drug causing vascular disorder. That is, it is an object of the present invention to provide: a composition capable of alleviate vascular disorder containing a drug causing vascular disorder; a solution for use in preparation of an administration solution containing a water-soluble drug causing vascular disorder, which is used for preparation of the aforementioned composition; a kit including a set of a water-soluble drug preparation causing vascular disorder and the solution for use in preparation of an administration solution containing a water-soluble drug; an agent for suppressing vascular disorder for a water-soluble drug causing vascular disorder; and a solution used in combination with a water-soluble drug causing vascular disorder, each of which is able to alleviate vascular disorder.

Means for Solving the Object

As a result of intensive studies directed towards achieving the aforementioned objects, the present inventors have found that a composition containing 1 part by mass or more of a nonionic surfactant with respect to 1 part by mass of a water-soluble drug causing vascular disorder significantly suppresses the factors causing vascular disorder, such as cytotoxicity.

Thus, the present invention relates to the following [1] to [20].

[1] A composition containing a water-soluble drug causing vascular disorder, wherein the composition comprises 1 part by mass or more of a nonionic surfactant with respect to 1 part by mass of the water-soluble drug causing vascular disorder.

[2] The composition containing a water-soluble drug causing vascular disorder according to [1], wherein the composition comprises 2 parts by mass or more of the nonionic surfactant with respect to 1 part by mass of the water-soluble drug causing vascular disorder.

[3] The composition containing a water-soluble drug causing vascular disorder according to [1] or [2], wherein the nonionic surfactant is one or more selected from the group consisting of polyoxyethylene castor oil (Cremophor), polysorbate, polyoxyethylene hydrogenated castor oil, and polyoxyethylene polyoxypropylene glycol.

[4] The composition containing a water-soluble drug causing vascular disorder according to any one of [1] to [3], which is a preparation containing a water-soluble drug causing vascular disorder.

[5] The composition containing a water-soluble drug causing vascular disorder according to any one of [1] to [3], which is an administration solution containing a water-soluble drug causing vascular disorder.

[6] A solution comprising a nonionic surfactant, for use in preparation of an administration solution containing a water-soluble drug causing vascular disorder, wherein the administration solution prepared using the solution containing a water-soluble drug causing vascular disorder prepared using the solution comprising the nonionic surfactant comprises 1 part by mass or more of the nonionic surfactant with respect to 1 part by mass of the water-soluble drug causing vascular disorder.

[7] The solution comprising a nonionic surfactant, for use in preparation of an administration solution containing a water-soluble drug causing vascular disorder according to [6], which is used to prepare an administration solution comprising a water-soluble drug causing vascular disorder, containing 2 parts by mass or more of the nonionic surfactant with respect to 1 part by mass of the water-soluble drug causing vascular disorder.

[8] The solution comprising a nonionic surfactant, for use in preparation of an administration solution containing a water-soluble drug causing vascular disorder according to [6] or [7], wherein the nonionic surfactant is one or more selected from the group consisting of polyoxyethylene castor oil (Cremophor), polysorbate, polyoxyethylene hydrogenated castor oil, and polyoxyethylene polyoxypropylene glycol.

[9] A kit including a set of a water-soluble drug preparation causing vascular disorder, and a solution comprising a nonionic surfactant, for use in preparation of an administration solution containing a water-soluble drug causing vascular disorder, wherein the administration solution containing a water-soluble drug causing vascular disorder that is prepared by dissolving the water-soluble drug preparation causing vascular disorder in the solution comprises 1 part by mass or more of the nonionic surfactant with respect to 1 part by mass of the water-soluble drug causing vascular disorder.

[10] The kit according to [9], wherein the administration solution containing a water-soluble drug causing vascular disorder that is prepared by dissolving the water-soluble drug preparation causing vascular disorder in the dissolving solution becomes an administration solution containing a water-soluble drug causing vascular disorder, comprising 2 parts by mass or more of the nonionic surfactant with respect to 1 part by mass of the water-soluble drug causing vascular disorder.

[11] The kit according to [9] or [10], wherein the nonionic surfactant is one or more selected from the group consisting of polyoxyethylene castor oil (Cremophor), polysorbate, polyoxyethylene hydrogenated castor oil, and polyoxyethylene polyoxypropylene glycol.

[12] An agent for suppressing vascular disorder for a water-soluble drug causing vascular disorder, wherein the agent for suppressing vascular disorder comprises a nonionic surfactant, wherein 1 part by mass or more of the nonionic surfactant is used with respect to 1 part by mass of the water-soluble drug causing vascular disorder.

[13] The agent for suppressing vascular disorder for a water-soluble drug causing vascular disorder, according to [12], wherein 2 parts by mass or more of the nonionic surfactant is used with respect to 1 part by mass of the water-soluble drug causing vascular disorder.

[14] The agent for suppressing vascular disorder, containing a water-soluble drug causing vascular disorder, according to [12] or [13], wherein the nonionic surfactant is one or more selected from the group consisting of polyoxyethylene castor oil (Cremophor), polysorbate, polyoxyethylene hydrogenated castor oil, and polyoxyethylene polyoxypropylene glycol.

[15] A solution containing a nonionic surfactant, which is used in combination with a water-soluble drug causing vascular disorder, wherein 1 part by mass or more of the nonionic surfactant is used with respect to 1 part by mass of the water-soluble drug causing vascular disorder.

[16] The solution according to [15], wherein 2 parts by mass or more of the nonionic surfactant is used with respect to 1 part by mass of the water-soluble drug causing vascular disorder.

[17] The solution according to [15] or [16], wherein the nonionic surfactant is one or more selected from the group consisting of polyoxyethylene castor oil (Cremophor), polysorbate, polyoxyethylene hydrogenated castor oil, and polyoxyethylene polyoxypropylene glycol.

[18] The composition containing a water-soluble drug causing vascular disorder, the solution for use in preparation of an administration solution containing a water-soluble drug causing vascular disorder, the kit, the agent for suppressing vascular disorder, or the solution according to any one of [1] to [17], wherein the water-soluble drug causing vascular disorder is a drug selected from the group consisting of fosaprepitant, a vinca alkaloid-based drug, and a platinum-based drug.

[19] The composition containing a water-soluble drug causing vascular disorder, the solution for use in preparation of an administration solution containing a water-soluble drug causing vascular disorder, the kit, the agent for suppressing vascular disorder, or the solution according to any one of [1] to [17], wherein the water-soluble drug causing vascular disorder is vinorelbine.

[20] The composition containing a water-soluble drug causing vascular disorder, the solution for use in preparation of an administration solution containing a water-soluble drug causing vascular disorder, the kit, the agent for suppressing vascular disorder, or the solution according to any one of [1] to [17], wherein the water-soluble drug causing vascular disorder is oxaliplatin.

Advantageous Effects of Invention

According to the present invention, vascular disorder caused by a water-soluble drug causing vascular disorder can be alleviated. Further, according to the present invention, it can be expected that the effect of reducing side-effect measures taken by healthcare professionals and the effect of improving the quality of life of patients can be obtained.

Brief Description of Drawings

[Fig. 1] Fig. 1 shows the results of the pathological scores of a blood vessel stimulation test using the posterior auricular vein of rabbits. The results are shown with a mean value of 2 rabbits in each group.
[Fig. 2] Fig. 2 shows the results of a histopathological examination in a blood vessel stimulation test using the posterior auricular vein of rabbits.

(A) Negative control (normal saline): no findings
(B) Comparative Example 1 (fosaprepitant normal saline aqueous solution): disappearance of vein endothelial cells 3+, thrombosis 3+, bleeding 2+, inflammatory cell infiltration 3+, and edema around vein 3+
(C) Example 1 (Comparative Example 1, containing about 7.2 parts by mass of a nonionic surfactant): disappearance of vein endothelial cells 1+, inflammatory cell infiltration 1+, and edema around vein 1+
(D) Example 4 (Comparative Example 1, containing about 2.0 parts by mass of a nonionic surfactant): disappearance of vein endothelial cells 2+, thrombosis 1+, inflammatory cell infiltration 1+, and edema around vein 1+
(E) Example 6 (Comparative Example 1, containing about 3.2 parts by mass of a nonionic surfactant): disappearance of vein endothelial cells 3+, thrombosis 1+, bleeding 1+, inflammatory cell infiltration 1+, and edema around vein 2+
(F) Example 7 (Comparative Example 1, containing about 2.0 parts by mass of a nonionic surfactant): disappearance of vein endothelial cells 2+, thrombosis 1+, inflammatory cell infiltration 1+, and edema around vein 1+

Grades - 0: no change; 1+: mild degree; 2+: moderate degree; 3+: strong degree; and 4+: severe degree

Embodiments of Carrying out the Invention

Hereinafter, specific embodiments of the present invention will be described in detail. However, the following embodiments are not intended to limit the scope of the present invention, and the present invention may be appropriately modified and may be carried out within the range of the purpose of one embodiment of the present invention.

< Composition containing water-soluble drug causing vascular disorder >

The composition of the present invention is characterized in that the composition contains 1 part by mass or more of a nonionic surfactant with respect to 1 part by mass of a water-soluble drug causing vascular disorder.
The water-soluble drug causing vascular disorder of the present invention is not particularly limited, as long as it is a drug or a pharmaceutically acceptable salt thereof, which can be dissolved in water and causes vascular disorder.

When such a water-soluble drug is, for example, an anticancer agent, examples of the drug may include: nucleic acid-based anticancer agents such as azacitidine, cladribine, and gemcitabine; anthracycline-based anticancer agents such as doxorubicin, epirubicin, and amrubicin; vinca alkaloid-based anticancer agents such as vinorelbine and vinblastine; platinum complex-based anticancer agents such as cisplatin and oxaliplatin; irinotecan, nogitecan, eribulin, pralatrexate, bendamustine, bortezomib, romidepsin, mitomycin C, and etoposide phosphate; and radioactive anticancer agents such as radium chloride and lutetium oxodotreotide. When the water-soluble drug is an antiemetic agent, examples of the water-soluble drug may include fosaprepitant and palonosetron. When the water-soluble drug is a therapeutic agent for bone lesion/hypercalcemia, an example of the water-soluble drug may be zoledronic acid. Other examples of the water-soluble drug may include: therapeutic agents for extravasation of anthracycline-based anticancer agents, such as dexrazoxane; therapeutic agents for febrile neutropenia, such as meropenem; therapeutic agents for osteoporosis, such as ibandronic acid; antiarrhythmic agents such as esmolol; analgesics such as oxycodone and hydromorphone; antidepressants such as clomipramine; antibacterial agents such as colistin, cefotaxime, ceftriaxone, daptomycin, tigecycline, tedizoliphosphate, pazufloxacin, micafungin, and levofloxacin; cyanide poisoning antidotes such as hydroxocobalamin; ethylene glycol/methanol poisoning antidotes, such as fomepizole; therapeutic agents for apnea of prematurity, such as caffeine citrate; and imaging agents such as florbetapyr.

Among the water-soluble drugs causing vascular disorder of the present invention, for example, fosaprepitant or a pharmaceutically acceptable salt thereof is an antiemetic agent having a selective NK1 receptor antagonistic action. Japanese Patent No. 3073770 discloses that fosaprepitant can form salts with various inorganic or organic acids and bases. Examples of such acid addition salts may include acetate, adipate, benzoate, benzenesulfonate, bisulfate, butyrate, citrate, camphorate, camphor sulfonate, ethane sulfonate, fumarate, hemisulfate, heptanoate, hexanoate, hydrochloride, hydrobromide, hydroiodide, methanesulfonate, lactate, maleate, methanesulfonate, 2-naphthalenesulfonate, oxalate, pamoate, persulfate, picrate, pivalate, propionate, citrate, succinate, tosylate and undecanoate. Examples of the basic salts may include: ammonium salts; alkaline metal salts such as sodium, lithium and potassium salts; alkaline earth metal salts such as calcium and magnesium salts; salts with organic bases, such as dicyclohexylamine salts; N-methyl-D-glucamine (meglumine); and salts with amino acids such as and arginine, lysine and ornithine. Fosaprepitant or a pharmaceutically acceptable salt thereof is a morpholine tachykinin receptor antagonist, and can be obtained according to the method described in Japanese Patent No. 3073770. Fosaprepitant or a pharmaceutically acceptable salt thereof is preferably of a quality level that allows it to be used as an active ingredient for pharmaceutical products. In the present invention, it is preferable to use fosaprepitant meglumine. Fosaprepitant meglumine is disclosed in Japanese Patent No. 3073770, and can be obtained according to the method described in Japanese Patent No. 3073770.

Among the water-soluble drugs causing vascular disorder of the present invention, examples of the vinca alkaloid-based anticancer agent may include vinorelbine, vinblastine, vincristine, and vindesine. The vinca alkaloid-based drug forms salts with various inorganic and organic acids, etc. The pharmaceutically acceptable salt thereof is not particularly limited, as long as it is of a quality level that allows it to be used as an active ingredient for pharmaceutical products. Preferred examples of such salts may include tartrate, hydrochloride, sulfate, and acetate.

Among the water-soluble drugs causing vascular disorder of the present invention, examples of the platinum complex-based anticancer agent may include oxaliplatin, cisplatin, and carboplatin.

The nonionic surfactant of the present invention is not particularly limited, as long as the present nonionic surfactant can be used as an additive for pharmaceutical products, suppresses the onset of vasculitis and angialgia caused by the water-soluble drug causing vascular disorder, and does not inhibit the water solubilization of the water-soluble drug causing vascular disorder. Examples of such a nonionic surfactant may include polyoxyethylene castor oil (Cremophor), polysorbate, polyoxyethylene hydrogenated castor oil, polyoxyethylene polyoxypropylene glycol (Poloxamer, registered trademark), polyoxyethylene sorbitan laurate, a polyethylene glycol-polylactic acid block polymer, a polyethylene glycol-poly(lactide-CO-glycolide) block polymer, a polyethylene glycol-poly(hydrophobic group-modified) amino acid block polymer, and a polyethylene glycol-polycaptolactone block polymer. The above-described nonionic surfactants may be used alone, or may also be used in combination of two or more types. In the present invention, the nonionic surfactant is preferably one or more selected from the group consisting of polyoxyethylene castor oil (Cremophor), polysorbate, polyoxyethylene hydrogenated castor oil, and polyoxyethylene polyoxypropylene glycol; is more preferably one or more selected from the group consisting of Cremophor, polysorbate, and polyoxyethylene hydrogenated castor oil; and is further preferably one or more selected from the group consisting of Cremophor, Polysorbate 80, and Polyoxyethylene Hydrogenated Castor Oil 60.

The amount of the nonionic surfactant contained in the composition of the present invention may be 1 part by mass or more with respect to 1 part by mass of the water-soluble drug causing vascular disorder, and the amount of the nonionic surfactant is more preferably larger than 1 part by mass, and is further preferably 2 parts by mass or more. The composition of the present invention preferably contains the nonionic surfactant in an amount of 2 parts by mass or more and 10 parts by mass or less, with respect to 1 part by mass of the water-soluble drug causing vascular disorder. In the composition of the present invention, as such a water-soluble drug causing vascular disorder, a water-soluble drug causing vascular disorder or a pharmaceutically acceptable salt thereof can be used. Herein, the amount of the nonionic surfactant indicates the amount thereof with respect to the amount of the water-soluble drug causing vascular disorder, from which counterion components are excluded. For example, in the case of a fosaprepitant preparation, when the composition comprises 245.3 mg of fosaprepitant meglumine, it means that 150 mg of fosaprepitant is comprised in the composition. That is to say, in order to achieve the composition of the present invention, the composition may comprise 1 part by mass or more of, namely, 150 mg or more of the nonionic surfactant, with respect to 150 mg of fosaprepitant.

By allowing the composition of the present invention to comprise 1 part by mass or more of the nonionic surfactant with respect to 1 part by mass of the water-soluble drug causing vascular disorder, vascular disorder occurring upon administration of the water-soluble drug causing vascular disorder can be alleviated. Specifically, vasculitis can be alleviated, and further, angialgia, regarding which vasculitis is considered to be one cause of the onset thereof, can also be alleviated. Furthermore, redness, pain, induration, erosion, blister, ulcer, necrosis and the like, which are caused by extravasation of the drug in vasculitis, can be alleviated. Vascular disorder caused by administration of the water-soluble drug causing vascular disorder is considered to take place due to tissue cell damage in the blood vessels near a site injected with a high-concentration drug solution. Hence, it is considered that administration of the nonionic surfactant would prevent the contact of the high-concentration drug with the tissues and the cells, so that the vascular disorder could be alleviated.

The form of the composition of the present invention containing a water-soluble drug causing vascular disorder is not particularly limited, as long as the composition contains a nonionic surfactant in an amount of 1 part by mass or more with respect to 1 part by mass of the water-soluble drug causing vascular disorder. The present composition may be in an embodiment that the nonionic surfactant has been added to the composition in advance in an amount necessary for formulation. Otherwise, the present composition may also be in an embodiment that the nonionic surfactant is attached, as a special dissolving solvent containing a necessary amount thereof, to the water-soluble drug preparation causing vascular disorder, and then, the nonionic surfactant is added to the water-soluble drug preparation causing vascular disorder upon the use thereof. Examples of the form of the composition of the present invention may include: formulations such as a freeze-dried formulation, a liquid formulation, and an RTU formulation, a solution wherein a freeze-dried formulation is dissolved; and an administration solution diluted in an infusion bag for administration.

The composition of the present invention containing a water-soluble drug causing vascular disorder may comprise additives such as a pH adjuster, a stabilizer, an isotonic agent, an excipient, and a surfactant other than the nonionic surfactant, as long as the composition suppresses the onset of vascular disorder caused by the water-soluble drug causing vascular disorder and does not inhibit water solubilization of the water-soluble drug preparation causing vascular disorder. In addition, the types of such additives may be of one type or may also be a mixture of two or more types.

Among such additives, examples of the pH adjuster may include acidic agents including inorganic acids such as hydrochloric acid, phosphoric acid, boric acid and carbonic acid, and organic acids such as ascorbic acid and acetic acid. Other examples of the pH adjuster may include alkaline agents including: the hydroxides of alkaline metals or alkaline earth metals, such as sodium hydroxide, potassium hydroxide, and lithium hydroxide; and the alkaline earth metal salts of inorganic acids, such as sodium dihydrogen phosphate, disodium monohydrogen phosphate, sodium carbonate, and sodium hydrogen carbonate. Moreover, buffers prepared by mixing the above-described acidic agents and alkaline agents with one another and then adjusting the pH thereof may also be used. The amount of the pH adjuster used can be appropriately adjusted, so that it can be prepared within the range of the above-described purpose.

Examples of the stabilizer may include ascorbic acid or a salt thereof, aspartic acid or a salt thereof, acetyltryptophan or a salt thereof, arginine or a salt thereof, sodium bisulfite, sodium sulfite, inositol, edetic acid or a salt thereof, erythorbic acid or a salt thereof, sodium chloride, fructose, xylitol, citric acid or a salt thereof, glycine, glycerin, gluconic acid or a salt thereof, glutamic acid or a salt thereof, creatinine, acetic acid or a salt thereof, cyclodextrin, cysteine or a salt thereof, tartaric acid or a salt thereof, sorbitol, thioglycolic acid or a salt thereof, thiosulfuric acid or a salt thereof, trometamol, lactic acid or a salt thereof, urea, sucrose, histidine or a salt thereof, pyrosulfurous acid or a salt thereof, glucose, propylene glycol, macrogol, methionine, lysine or a salt thereof, phosphoric acid or a salt thereof, and leucine.

Examples of the isotonic agent may include sodium chloride, lactose, sucrose, inositol, fructose, glucose, glycine, glycerin, sorbitol, xylitol, nicotinamide, macrogol, propylene glycol, and pyrophosphoric acid or a salt thereof.

Examples of the excipient may include sodium chloride, lactose, sucrose, inositol, fructose, glucose, glycine, glycerin, gluconic acid or a salt thereof, cyclodextrin, tartaric acid or a salt thereof, citric acid or a salt thereof, and macrogol.

Examples of the surfactant other than the nonionic acid may include: anionic surfactants such as sodium desoxycholate, sodium ursodesoxycholate, and sodium lauryl sulfate; cationic surfactants such as benzethonium chloride; and lecithin.

The preparation of the present invention containing a water-soluble drug causing vascular disorder is characterized in that the preparation contains a nonionic surfactant in an amount of 1 part by mass or more with respect to 1 part by mass of the water-soluble drug causing vascular disorder. The present invention is able to alleviate vascular disorder occurring upon administration of the water-soluble drug causing vascular disorder.

The preparation of the present invention containing a water-soluble drug causing vascular disorder contains a nonionic surfactant in an amount of 1 part by mass or more with respect to 1 part by mass of the water-soluble drug causing vascular disorder, and is in the form of a preparation such as a freeze-dried formulation, a liquid formulation, or an RTU formulation. In the present invention, the preferred nonionic surfactant and the amount thereof are the same as those described above.

The preparation of the present invention containing a water-soluble drug causing vascular disorder may comprise other additives used in common pharmaceutical preparations, such as a pH adjuster, a surfactant, a stabilizer, an isotonic agent, an excipient, and a surfactant other than the nonionic surfactant, in addition to the water-soluble drug causing vascular disorder and the nonionic surfactant. In the present invention, the additives that may be comprised in the present preparation are the same as those described above.

< Solution for use in preparation of administration solution containing water-soluble drug causing vascular disorder >

The solution of the present invention for use in preparation of an administration solution containing a water-soluble drug causing vascular disorder comprises a nonionic surfactant. With regard to the solution of the present invention for use in preparation of an administration solution containing a water-soluble drug causing vascular disorder, the administration solution containing a water-soluble drug causing vascular disorder that is prepared using the above-described solution containing a nonionic surfactant comprises the above-described nonionic surfactant in an amount of 1 part by mass or more with respect to 1 part by mass of the water-soluble drug causing vascular disorder. The present invention is able to alleviate vascular disorder occurring upon administration of the water-soluble drug causing vascular disorder.

The present solution of the present invention for use in preparation of an administration solution containing a water-soluble drug causing vascular disorder is preferably a solution for use in preparation of an administration solution that is diluted for administration. In the present invention, the preferred nonionic surfactant and the amount thereof are the same as those described above.

In the present invention, examples of the solvent used for the dilution may include water for injection, a normal saline, and a 5% glucose aqueous solution. In addition, these solvents may comprise the aforementioned nonionic surfactant.

The solution of the present invention for use in preparation of an administration solution containing a water-soluble drug causing vascular disorder is characterized in that the administration solution containing a water-soluble drug causing vascular disorder that is prepared using the solution of the present invention contains a nonionic surfactant in an amount of 1 part by mass or more with respect to 1 part by mass of the water-soluble drug causing vascular disorder. The present invention is able to alleviate vascular disorder occurring upon administration of the water-soluble drug causing vascular disorder. In the case of administration of a water-soluble drug preparation causing vascular disorder, wherein the amount of a nonionic surfactant is less than 1 part by mass with respect to 1 part by mass of the water-soluble drug causing vascular disorder, the preparation is dissolved in the solution of the present invention for use in preparation of an administration solution containing a water-soluble drug causing vascular disorder, which contains a nonionic surfactant, so that the amount of the nonionic surfactant in the solution becomes 1 part by mass or more with respect to 1 part by mass of the water-soluble drug causing vascular disorder. Thereby, vascular disorder occurring upon administration of the water-soluble drug causing vascular disorder can be alleviated. Examples of such a water-soluble drug preparation causing vascular disorder, wherein the amount of a nonionic surfactant is less than 1 part by mass with respect to 1 part by mass of the water-soluble drug causing vascular disorder, may include the existing fosaprepitant preparations such as a fosaprepitant meglumine for intravenous infusion preparation PROEMEND (registered trademark) and a preparation produced according to the method described in JP Patent Publication (Kokai) No. 2019-73457 A. The above-described fosaprepitant meglumine intravenous drip preparation PROEMEND (registered trademark) and the above-described preparation produced according to the method described in JP Patent Publication (Kokai) No. 2019-73457 A each contain 0.5 parts by mass of the nonionic surfactant (Polysorbate 80) with respect to 1 part by mass of fosaprepitant.

The amount of the nonionic surfactant contained in the solution of the present invention for use in preparation of an administration solution containing a water-soluble drug causing vascular disorder may be set, so that the prepared administration solution containing a water-soluble drug causing vascular disorder contains the nonionic surfactant in an amount of 1 part by mass or more with respect to 1 part by mass of the water-soluble drug causing vascular disorder. The amount of the nonionic surfactant is preferably 2 parts by mass and is preferably 2 parts by mass or more and 10 parts by mass or less, with respect to 1 part by mass of the water-soluble drug causing vascular disorder.

The administration solution containing a water-soluble drug causing vascular disorder prepared using the solution of the present invention for use in preparation of an administration solution containing a water-soluble drug causing vascular disorder is an administration solution containing a water-soluble drug causing vascular disorder containing a nonionic surfactant in an amount of preferably 2 parts by mass or more, and preferably 2 parts by mass or more and 10 parts by mass or less, with respect to 1 part by mass of the water-soluble drug causing vascular disorder. In the present invention, the preferred nonionic surfactants are the same as those described above.

The solution of the present invention for use in preparation of an administration solution containing a water-soluble drug causing vascular disorder may comprise a solvent. Examples of the solvent that may be comprised in the solution of the present invention may include water for injection, a normal saline, and a 5% glucose aqueous solution.

The solution of the present invention for use in preparation of an administration solution containing a water-soluble drug causing vascular disorder may comprise a pH adjuster and the like, in addition to the nonionic surfactant and the solvent. In addition, such additives may be of one type, or may also be a mixture of two or more types. Among such additives, examples of the pH adjuster may include acidic agents including inorganic acids such as hydrochloric acid, phosphoric acid, boric acid and carbonic acid, and organic acids such as ascorbic acid and acetic acid. Other examples of the pH adjuster may include alkaline agents including: the hydroxides of alkaline metals or alkaline earth metals, such as sodium hydroxide, potassium hydroxide, and lithium hydroxide; and the alkaline earth metal salts of inorganic acids, such as sodium dihydrogen phosphate, disodium monohydrogen phosphate, sodium carbonate, and sodium hydrogen carbonate. Moreover, buffers prepared by mixing the above-described acidic agents and alkaline agents with one another and then adjusting the pH thereof may also be used. The amount of the pH adjuster used can be appropriately adjusted, so that it can be prepared within the range of the above-described purpose. Among the additives other than the surfactant, examples of the stabilizer may include ascorbic acid or a salt thereof, aspartic acid or a salt thereof, acetyltryptophan or a salt thereof, arginine or a salt thereof, sodium bisulfite, sodium sulfite, inositol, edetic acid or a salt thereof, erythorbic acid or a salt thereof, sodium chloride, fructose, xylitol, citric acid or a salt thereof, glycine, glycerin, gluconic acid or a salt thereof, glutamic acid or a salt thereof, creatinine, acetic acid or a salt thereof, cyclodextrin, cysteine or a salt thereof, tartaric acid or a salt thereof, sorbitol, thioglycolic acid or a salt thereof, thiosulfuric acid or a salt thereof, trometamol, lactic acid or a salt thereof, urea, sucrose, histidine or a salt thereof, pyrosulfurous acid or a salt thereof, glucose, propylene glycol, macrogol, methionine, lysine or a salt thereof, phosphoric acid or a salt thereof, and leucine. Moreover, among the additives other than the surfactant, examples of the isotonic agents may include sodium chloride, lactose, sucrose, inositol, fructose, glucose, glycine, glycerin, sorbitol, xylitol, nicotinamide, macrogol, propylene glycol, and pyrophosphoric acid or a salt thereof.

The form of the solution of the present invention for use in preparation of an administration solution containing a water-soluble drug causing vascular disorder may be a special solution wherein the water-soluble drug preparation causing vascular disorder is dissolved or may also be a solution filled in an infusion solution bag for administration.

<Kit>

According to the present invention, provided is a kit including a set of a water-soluble drug preparation causing vascular disorder, and a solution for use in preparation of an administration solution containing a water-soluble drug causing vascular disorder, comprising a nonionic surfactant.

The kit of the present invention including a set of a water-soluble drug preparation causing vascular disorder and a nonionic surfactant-containing solution is a kit, wherein the water-soluble drug preparation causing vascular disorder is dissolved in the above-described solution, so as to obtain an administration solution containing a water-soluble drug causing vascular disorder, which comprises the nonionic surfactant in an amount of 1 part by mass or more with respect to 1 part by mass of the water-soluble drug causing vascular disorder. By allowing the kit to include a set of the water-soluble drug preparation causing vascular disorder and the nonionic surfactant-containing solution, the prescription of the water-soluble drug preparation causing vascular disorder does not need to be changed, and thus, there is no need to worry about a reduction in preservation stability due to the prescription changes, so that a necessary amount of the nonionic surfactant can be added at the time of use.

The administration solution containing a water-soluble drug causing vascular disorder prepared using the kit of the present invention including a set of the water-soluble drug preparation causing vascular disorder and the nonionic surfactant-containing solution is an administration solution containing a water-soluble drug causing vascular disorder, which contains the nonionic surfactant in an amount of, preferably 2 parts by mass or more, and preferably 2 parts by mass or more and 10 parts by mass or less, with respect to 1 part by mass of the water-soluble drug causing vascular disorder. In the present invention, the preferred nonionic surfactants are the same as those described above.

The water-soluble drug preparation of the present invention causing vascular disorder is not particularly limited, as long as it is a water-soluble drug preparation causing vascular disorder, wherein the amount of the nonionic surfactant is less than 1 part by mass with respect to 1 part by mass of the water-soluble drug causing vascular disorder, and it is a commonly used water-soluble drug preparation causing vascular disorder. For example, in the case of a fosaprepitant preparation, examples of the preparation may include the existing fosaprepitant preparation, namely, a fosaprepitant meglumine for intravenous infusion preparation PROEMEND (registered trademark), and a preparation produced according to the method described in JP Patent Publication (Kokai) No. 2019-73457 A.

< Agent for suppressing vascular disorder >

The agent of the present invention for suppressing vascular disorder for a water-soluble drug causing vascular disorder, comprises a nonionic surfactant, and uses the nonionic surfactant in an amount of 1 part by mass or more with respect to 1 part by mass of the water-soluble drug causing vascular disorder. Vascular disorder caused by administration of the water-soluble drug causing vascular disorder is considered to occur due to tissue cell damage in the blood vessels near a site injected with a high-concentration drug solution. Hence, it is considered that administration of the agent for suppressing vascular disorder for a water-soluble drug causing vascular disorder, which comprises the nonionic surfactant, would prevent the contact of the high-concentration drug with the tissues and the cells, so that the vascular disorder could be alleviated. The agent for suppressing vasculitis and angialgia caused by a water-soluble drug causing vascular disorder may be administered, as an administration solution containing a water-soluble drug causing vascular disorder, simultaneously with the water-soluble drug preparation causing vascular disorder, or may also be administered before administration of the water-soluble drug preparation causing vascular disorder (pre-administration).

The agent of the present invention for suppressing vascular disorder for a water-soluble drug causing vascular disorder, uses the nonionic surfactant in an amount of, preferably 2 parts by mass or more, and preferably 2 parts by mass or more and 10 parts by mass or less, with respect to 1 part by mass of the water-soluble drug causing vascular disorder. In the present invention, the preferred nonionic surfactants are the same as those described above. When the water-soluble drug preparation causing vascular disorder to be administered contains 0.5 parts by mass of the nonionic surfactant with respect to 1 part by mass of the water-soluble drug causing vascular disorder, the amount of the nonionic surfactant contained in fosaprepitant that is the agent of the present invention for suppressing vascular disorder may be 0.5 parts by mass or more. When the water-soluble drug preparation causing vascular disorder contains 0.5 parts by mass of the nonionic surfactant with respect to 1 part by mass of the water-soluble drug causing vascular disorder, the amount of the nonionic surfactant contained in the agent of the present invention for suppressing vascular disorder containing a water-soluble drug causing vascular disorder for a water-soluble drug causing vascular disorder, is preferably 1.5 parts by mass or more, and preferably 1.5 parts by mass or more and 9.5 parts by mass or less.

< Solution containing a nonionic surfactant >

The solution of the present invention containing a nonionic surfactant can be used in combination with the water-soluble drug causing vascular disorder. In the present description, the phrase "to use in combination with ..." means that the solution of the present invention containing a nonionic surfactant is administered simultaneously with the water-soluble drug causing vascular disorder, or that the solution of the present invention containing a nonionic surfactant is administered before administration of the water-soluble drug causing vascular disorder.

In the solution of the present invention containing a nonionic surfactant, the nonionic surfactant is used in an amount of 1 part by mass or more, preferably 2 parts by mass or more, and preferably 2 parts by mass or more and 10 parts by mass or less, with respect to 1 part by mass of the water-soluble drug causing vascular disorder. In the present invention, the preferred nonionic surfactants are the same as those described above. When the water-soluble drug preparation causing vascular disorder to be administered contains 0.5 parts by mass of the nonionic surfactant with respect to 1 part by mass of the water-soluble drug causing vascular disorder, the amount of the nonionic surfactant contained in the solution of the present invention containing a nonionic surfactant may be 0.5 parts by mass or more. When the water-soluble drug preparation causing vascular disorder to be administered contains 0.5 parts by mass of the nonionic surfactant with respect to 1 part by mass of the water-soluble drug causing vascular disorder, the amount of the nonionic surfactant contained in the solution of the present invention containing a nonionic surfactant is preferably 1.5 parts by mass or more, and preferably 1.5 parts by mass or more and 9.5 parts by mass or less.

Examples

Hereinafter, the present invention will be more specifically described in the following examples regarding fosaprepitant. However, these examples are not intended to limit the scope of the present invention. It is to be noted that the fosaprepitant preparation used in the following examples and comparative examples is a freeze-dried formulation produced by the method described in JP Patent Publication (Kokai) No. 2019-73457 A, and specifically, it is a fosaprepitant preparation produced using 257.6 mg of fosaprepitant meglumine (157.5 mg of fosaprepitant), 5.7 mg of disodium edetate hydrate, 78.8 mg of Polysorbate 80, 393.8 mg of maltose hydrate, an appropriate amount of sodium hydroxide, and an appropriate amount of hydrochloric acid. As with PROEMEND (product name) as a fosaprepitant preparation, the fosaprepitant preparation used in the examples and the comparative example contained 157.5 mg of fosaprepitant and 78.8 mg of Polysorbate 80 (since the 150 mg PROEMEND product is 5% overfilled with fosaprepitant). Besides, the amount of Polysorbate 80 in the above-described fosaprepitant preparation was 0.5 parts by mass with respect to 1 part by mass of fosaprepitant.

[Example 1] Production of fosaprepitant solution by addition of Cremophor to fosaprepitant preparation in amount of about 6.7 parts by mass (1000 mg) with respect to 1 part by mass of fosaprepitant (containing about 7.2 parts by mass of nonionic surfactants including Polysorbate 80 contained in preparation)

1174 mg (about 1.12 mL) of Cremophor (Kolliphor ELP, manufactured by Nippon Oil & Fats Co., Ltd.) was added to 4.75 mL of a normal saline (manufactured by Otsuka Pharmaceutical Co., Ltd.), and the obtained mixture was stirred at room temperature for 10 minutes, and was then left at rest for 2 hours to produce a solution. 5 mL of the produced solution (containing about 1000 mg of Cremophor; about 6.7 parts by mass with respect to 1 part by mass of fosaprepitant) was weighed with a syringe and was then added to a fosaprepitant preparation, and thereafter, the obtained mixture was gently stirred for 10 minutes, so as to produce a fosaprepitant solution (containing 30 mg/mL fosaprepitant). The pH of the fosaprepitant solution produced by the present method was pH 7.7. The produced solution was maintained in the state of an almost transparent solution at room temperature or lower for 1 day or more.

[Example 2] Production of fosaprepitant solution by addition of Cremophor to fosaprepitant preparation in amount of about 3.3 parts by mass (500 mg) with respect to 1 part by mass of fosaprepitant (containing about 3.8 parts by mass of nonionic surfactants including Polysorbate 80 contained in preparation)

634 mg (about 0.60 mL) of Cremophor (Kolliphor ELP, manufactured by Nippon Oil & Fats Co., Ltd.) was added to 5.74 mL of a normal saline (manufactured by Otsuka Pharmaceutical Co., Ltd.), and the obtained mixture was stirred at room temperature for 10 minutes, and was then left at rest for 2 hours to produce a solution. 5 mL of the produced solution (containing about 500 mg of Cremophor; about 3.3 parts by mass with respect to 1 part by mass of fosaprepitant) was weighed with a syringe and was then added to a fosaprepitant preparation, and thereafter, the obtained mixture was gently stirred for 10 minutes, so as to produce a fosaprepitant solution (containing 30 mg/mL fosaprepitant). The pH of the fosaprepitant solution produced by the present method was pH 8.0. The produced solution was maintained in the state of an almost transparent solution at room temperature or lower for 1 day or more.

[Example 3] Production of fosaprepitant solution by addition of Cremophor to fosaprepitant preparation in amount of about 2.5 parts by mass (375 mg) with respect to 1 part by mass of fosaprepitant (containing about 3.0 parts by mass of nonionic surfactants including Polysorbate 80 contained in preparation)

541 mg (about 0.51 mL) of Cremophor (Kolliphor ELP, manufactured by Nippon Oil & Fats Co., Ltd.) was added to 6.69 mL of a normal saline (manufactured by Otsuka Pharmaceutical Co., Ltd.), and the obtained mixture was stirred at room temperature for 10 minutes, and was then left at rest for 2 hours to produce a solution. 5 mL of the produced solution (containing about 375 mg of Cremophor; about 2.5 parts by mass with respect to 1 part by mass of fosaprepitant) was weighed with a syringe and was then added to a fosaprepitant preparation, and thereafter, the obtained mixture was gently stirred for 10 minutes, so as to produce a fosaprepitant solution (containing 30 mg/mL fosaprepitant). The pH of the fosaprepitant solution produced by the present method was pH 8.1. The produced solution was maintained in the state of an almost transparent solution at room temperature or lower for 1 day or more.

[Example 4] Production of fosaprepitant solution by addition of Cremophor to fosaprepitant preparation in amount of about 1.5 parts by mass (225 mg) with respect to 1 part by mass of fosaprepitant (containing about 2.0 parts by mass of nonionic surfactants including Polysorbate 80 contained in preparation)

292 mg (about 0.28 mL) of Cremophor (Kolliphor ELP, manufactured by Nippon Oil & Fats Co., Ltd.) was added to 6.20 mL of a normal saline (manufactured by Otsuka Pharmaceutical Co., Ltd.), and the obtained mixture was stirred at room temperature for 10 minutes, and was then left at rest for 2 hours to produce a solution. 5 mL of the produced solution (containing about 225 mg of Cremophor; about 1.5 parts by mass with respect to 1 part by mass of fosaprepitant) was weighed with a syringe and was then added to a fosaprepitant preparation, and thereafter, the obtained mixture was gently stirred for 10 minutes, so as to produce a fosaprepitant solution (containing 30 mg/mL fosaprepitant). The pH of the fosaprepitant solution produced by the present method was pH 8.2. The produced solution was maintained in the state of an almost transparent solution at room temperature or lower for 1 day or more.

[Example 5] Production of fosaprepitant solution by addition of Cremophor to fosaprepitant preparation in amount of about 0.5 parts by mass (75 mg) with respect to 1 part by mass of fosaprepitant (containing about 1.0 part by mass of nonionic surfactants including Polysorbate 80 contained in preparation)

106 mg (about 0.10 mL) of Cremophor (Kolliphor ELP, manufactured by Nippon Oil & Fats Co., Ltd.) was added to 6.97 mL of a normal saline (manufactured by Otsuka Pharmaceutical Co., Ltd.), and the obtained mixture was stirred at room temperature for 10 minutes, and was then left at rest for 2 hours to produce a solution. 5 mL of the produced solution (containing about 75 mg of Cremophor; about 0.5 parts by mass with respect to 1 part by mass of fosaprepitant) was weighed with a syringe and was then added to a fosaprepitant preparation, and thereafter, the obtained mixture was gently stirred for 10 minutes, so as to produce a fosaprepitant solution (containing 30 mg/mL fosaprepitant). The pH of the fosaprepitant solution produced by the present method was pH 8.3. The produced solution was maintained in the state of an almost transparent solution at room temperature or lower for 1 day or more.

[Example 6] Production of fosaprepitant solution by addition of Polysorbate 80 to fosaprepitant preparation in amount of about 2.7 parts by mass (400 mg) with respect to 1 part by mass of fosaprepitant (containing about 3.2 parts by mass of nonionic surfactants including that contained in preparation)

584 mg (about 0.55 mL) of Polysorbate 80 (manufactured by KANTO CHEMICAL CO., INC.) was added to 6.75 mL of a normal saline (manufactured by Otsuka Pharmaceutical Co., Ltd.), and the obtained mixture was stirred at room temperature for 10 minutes and was then left at rest for 2 hours to produce a solution. 5 mL of the produced solution (containing about 400 mg of Polysorbate 80; about 2.7 parts by mass with respect to 1 part by mass of fosaprepitant) was weighed with a syringe and was then added to a fosaprepitant preparation, and thereafter, the obtained mixture was gently stirred for 10 minutes, so as to produce a fosaprepitant solution (containing 30 mg/mL fosaprepitant). The pH of the fosaprepitant solution produced by the present method was pH 8.1. The produced solution was maintained in the state of a transparent solution at room temperature or lower for 1 day or more.

[Example 7] Production of fosaprepitant solution by addition of Polysorbate 80 to fosaprepitant preparation in amount of about 1.5 parts by mass (225 mg) with respect to 1 part by mass of fosaprepitant (containing about 2.0 parts by mass of nonionic surfactants including that contained in preparation)

310 mg (about 0.29 mL) of Polysorbate 80 (manufactured by KANTO CHEMICAL CO., INC.) was added to 6.61 mL of a normal saline (manufactured by Otsuka Pharmaceutical Co., Ltd.), and the obtained mixture was stirred at room temperature for 10 minutes and was then left at rest for 2 hours to produce a solution. 5 mL of the produced solution (containing about 225 mg of Polysorbate 80; about 1.5 parts by mass with respect to 1 part by mass of fosaprepitant) was weighed with a syringe and was then added to a fosaprepitant preparation, and thereafter, the obtained mixture was gently stirred for 10 minutes, so as to produce a fosaprepitant solution (containing 30 mg/mL fosaprepitant). The pH of the fosaprepitant solution produced by the present method was pH 8.2. The produced solution was maintained in the state of a transparent solution at room temperature or lower for 1 day or more.

[Example 8] Production of fosaprepitant solution by addition of Polysorbate 80 to fosaprepitant preparation in amount of about 0.5 parts by mass (75 mg) with respect to 1 part by mass of fosaprepitant (containing about 1.0 part by mass of nonionic surfactants including that contained in preparation)

108 mg (about 0.10 mL) of Polysorbate 80 (manufactured by KANTO CHEMICAL CO., INC.) was added to 7.07 mL of a normal saline (manufactured by Otsuka Pharmaceutical Co., Ltd.), and the obtained mixture was stirred at room temperature for 10 minutes and was then left at rest for 2 hours to produce a solution. 5 mL of the produced solution (containing about 75 mg of Polysorbate 80; about 0.5 parts by mass with respect to 1 part by mass of fosaprepitant) was weighed with a syringe and was then added to a fosaprepitant preparation, and thereafter, the obtained mixture was gently stirred for 10 minutes, so as to produce a fosaprepitant solution (containing 30 mg/mL fosaprepitant). The pH of the fosaprepitant solution produced by the present method was pH 8.3. The produced solution was maintained in the state of a transparent solution at room temperature or lower for 1 day or more.

[Example 9] Production of fosaprepitant solution by addition of Polyoxyethylene Hydrogenated Castor Oil 60 to fosaprepitant preparation in amount of about 3.3 parts by mass (500 mg) with respect to 1 part by mass of fosaprepitant (containing about 3.8 parts by mass of nonionic surfactants including that contained in preparation)

1001 mg of Polyoxyethylene Hydrogenated Castor Oil 60 (manufactured by Nikko Chemicals Co., Ltd.) was added to about 5 mL of a normal saline (manufactured by Otsuka Pharmaceutical Co., Ltd.), and the obtained mixture was then stirred at 40°C for 10 minutes for dissolution. Thereafter, the amount of the solution was adjusted to 10 mL with a normal saline, and the obtained solution was then left at rest at room temperature for 2 hours to produce a solution. 5 mL of the produced solution (containing about 500 mg of Polyoxyethylene Hydrogenated Castor Oil 60; about 3.3 parts by mass with respect to 1 part by mass of fosaprepitant) was weighed with a syringe and was then added to a fosaprepitant preparation, and thereafter, the obtained mixture was gently stirred for 10 minutes, so as to produce a fosaprepitant solution (containing 30 mg/mL fosaprepitant). The pH of the fosaprepitant solution produced by the present method was pH 6.8. The produced solution was maintained in the state of a transparent solution at room temperature or lower for 1 day or more.

[Comparative Example 1] Production of fosaprepitant solution by dissolution of fosaprepitant preparation in normal saline

5 mL of a normal saline (manufactured by Otsuka Pharmaceutical Co., Ltd.) was weighed with a syringe and was then added to a fosaprepitant preparation, and thereafter, the obtained mixture was gently stirred for 10 minutes, so as to produce a fosaprepitant solution (containing 30 mg/mL fosaprepitant). The pH of the fosaprepitant solution produced by the present method was pH 8.4. The produced solution was maintained in the state of a transparent solution at room temperature or lower for 1 day or more.

[Comparative Example 2] Production of fosaprepitant solution by dissolution of fosaprepitant preparation in normal saline and hydrochloric acid

0.25 mL of 0.1 N hydrochloric acid water (manufactured by KANTO CHEMICAL CO., INC.; for use in volume analysis) was added to 5.75 mL of a normal saline (manufactured by Otsuka Pharmaceutical Co., Ltd.), and the obtained mixture was then left at rest for 2 hours to produce a solution. 5 mL of the produced solution (containing 0.21 mL of 0.1 N hydrochloric acid water) was weighed with a syringe and was then added to a fosaprepitant preparation, and thereafter, the obtained mixture was gently stirred for 10 minutes, so as to produce a fosaprepitant solution (containing 30 mg/mL fosaprepitant). The pH of the fosaprepitant solution produced by the present method was pH 7.8. The produced solution was maintained in the state of a transparent solution at room temperature or lower for 1 day or more.

[Comparative Example 3] Production of fosaprepitant solution by addition of Macrogol 300 to fosaprepitant preparation in amount of about 6.7 parts by mass (1000 mg) with respect to 1 part by mass of fosaprepitant

1236 mg (about 1.11 mL) of Macrogol 300 (manufactured by Nippon Oil & Fats Co., Ltd.) was added to 5.06 mL of a normal saline (manufactured by Otsuka Pharmaceutical Co., Ltd.), and the obtained mixture was stirred at room temperature for 10 minutes and was then left at rest for 2 hours to produce a solution. 5 mL of the produced solution (containing about 1000 mg Macrogol 300; about 6.7 parts by mass with respect to 1 part by mass of fosaprepitant) was weighed with a syringe and was then added to a fosaprepitant preparation, and thereafter, the obtained mixture was gently stirred for 10 minutes, so as to produce a fosaprepitant solution (containing 30 mg/mL fosaprepitant). The pH of the fosaprepitant solution produced by the present method was pH 8.2. The produced solution was maintained in the state of an almost transparent solution at room temperature or lower for 1 day or more.

Hereinafter, the present invention will be more specifically described in the following examples regarding vinorelbine. However, these examples are not intended to limit the scope of the present invention. It is to be noted that the vinorelbine preparation used in the following examples and the following comparative example is Rozeus Intravenous Injection 40 mg (product name), which is a preparation containing 55.4 mg of vinorelbine tartrate in 4 mL of a solution. The concentration of vinorelbine in this preparation is 10 mg/mL.

[Example X-1] Production of vinorelbine solution by addition of Cremophor in amount of about 10 parts by mass with respect to 1 part by mass of vinorelbine

0.076 mL (about 79.8 mg) of Cremophor (Kolliphor ELP, manufactured by Nippon Oil & Fats Co., Ltd.) was dissolved in 3.924 mL of a medium for Balb/3T3 cells (Dulbecco's Modified Eagle Medium (manufactured by Thermo Fisher) containing 10% fetal bovine serum (manufactured by Corning International) and a 100 units/mL penicillin - 100 $\mu$g/mL streptomycin solution (FUJIFILM Wako Pure Chemical Corporation) (hereinafter abbreviated as a "medium"), so as to produce a medium solution of Cremophor. The concentration of Cremophor in the solution was about 20 mg/mL.

0.01 mL of a vinorelbine preparation was extracted, and thereafter, 0.05 mL of the produced Cremophor solution and 0.94 mL of the medium were then added to the vinorelbine preparation. The obtained mixture was stirred at room temperature for 1 minute to produce a vinorelbine solution (containing 0.1 mg/mL vinorelbine). Moreover, 0.025 mL of a vinorelbine preparation was extracted, and thereafter, 0.125 mL of the produced Cremophor solution and 0.85 mL of the medium were then added to the vinorelbine preparation. The obtained mixture was stirred at room temperature for 1 minute to produce a vinorelbine solution (containing 0.25 mg/mL vinorelbine). Furthermore, 0.05 mL of a vinorelbine preparation was extracted, and thereafter, 0.25 mL of the produced Cremophor solution and 0.7 mL of the medium were then added to the vinorelbine preparation. The obtained mixture was stirred at room temperature for 1 minute to produce a vinorelbine solution (containing 0.5 mg/mL vinorelbine).

[Example X-2] Production of vinorelbine solution by addition of Cremophor in amount of about 2 parts by mass with respect to 1 part by mass of vinorelbine

0.01 mL of a vinorelbine preparation was extracted, and thereafter, 0.01 mL of the Cremophor solution produced in Example X-1 and 0.98 mL of the medium were then added to the vinorelbine preparation. The obtained mixture was stirred at room temperature for 1 minute to produce a vinorelbine solution (containing 0.1 mg/mL vinorelbine). Moreover, 0.025 mL of a vinorelbine preparation was extracted, and thereafter, 0.025 mL of the Cremophor solution produced in Example X-1 and 0.95 mL of the medium were then added to the vinorelbine preparation. The obtained mixture was stirred at room temperature for 1 minute to produce a vinorelbine solution (containing 0.25 mg/mL vinorelbine). Furthermore, 0.05 mL of a vinorelbine preparation was extracted, and thereafter, 0.05 mL of the Cremophor solution produced in Example X-1 and 0.9 mL of the medium were then added to the vinorelbine preparation. The obtained mixture was stirred at room temperature for 1 minute to produce a vinorelbine solution (containing 0.5 mg/mL vinorelbine).

[Example X-3] Production of vinorelbine solution by addition of Cremophor in amount of about 1 part by mass with respect to 1 part by mass of vinorelbine

0.01 mL of a vinorelbine preparation was extracted, and thereafter, 0.005 mL of the Cremophor solution produced in Example X-1 and 0.985 mL of the medium were then added to the vinorelbine preparation. The obtained mixture was stirred at room temperature for 1 minute to produce a vinorelbine solution (containing 0.1 mg/mL vinorelbine). Moreover, 0.025 mL of a vinorelbine preparation was extracted, and thereafter, 0.0125 mL of the Cremophor solution produced in Example X-1 and 0.9625 mL of the medium were then added to the vinorelbine preparation. The obtained mixture was stirred at room temperature for 1 minute to produce a vinorelbine solution (containing 0.25 mg/mL vinorelbine). Furthermore, 0.05 mL of a vinorelbine preparation was extracted, and thereafter, 0.025 mL of the Cremophor solution produced in Example X-1 and 0.925 mL of the medium were then added to the vinorelbine preparation. The obtained mixture was stirred at room temperature for 1 minute to produce a vinorelbine solution (containing 0.5 mg/mL vinorelbine).

[Example X-4] Production of vinorelbine solution by addition of Polysorbate 80 in amount of about 10 parts by mass with respect to 1 part by mass of vinorelbine

0.038 mL (about 40.4 mg) of Polysorbate 80 (manufactured by KANTO CHEMICAL CO., INC.) was dissolved in 3.962 mL of the medium, so as to produce a medium solution of Polysorbate 80. The concentration of Polysorbate 80 in the solution was about 10 mg/mL. 0.01 mL of a vinorelbine preparation was extracted, and thereafter, 0.1 mL of the produced Polysorbate 80 solution and 0.89 mL of the medium were then added to the vinorelbine preparation. The obtained mixture was stirred at room temperature for 1 minute to produce a vinorelbine solution (containing 0.1 mg/mL vinorelbine). Moreover, 0.025 mL of a vinorelbine preparation was extracted, and thereafter, 0.25 mL of the produced Polysorbate 80 solution and 0.725 mL of the medium were then added to the vinorelbine preparation. The obtained mixture was stirred at room temperature for 1 minute to produce a vinorelbine solution (containing 0.25 mg/mL vinorelbine). Furthermore, 0.05 mL of a vinorelbine preparation was extracted, and thereafter, 0.5 mL of the produced Polysorbate 80 solution and 0.45 mL of the medium were then added to the vinorelbine preparation. The obtained mixture was stirred at room temperature for 1 minute to produce a vinorelbine solution (containing 0.5 mg/mL vinorelbine).

[Example X-5] Production of vinorelbine solution by addition of Polysorbate 80 in amount of about 2 parts by mass with respect to 1 part by mass of vinorelbine

0.01 mL of a vinorelbine preparation was extracted, and thereafter, 0.02 mL of the Polysorbate 80 solution produced in Example X-4 and 0.97 mL of the medium were then added to the vinorelbine preparation. The obtained mixture was stirred at room temperature for 1 minute to produce a vinorelbine solution (containing 0.1 mg/mL vinorelbine). Moreover, 0.025 mL of a vinorelbine preparation was extracted, and thereafter, 0.05 mL of the Polysorbate 80 solution produced in Example X-4 and 0.925 mL of the medium were then added to the vinorelbine preparation. The obtained mixture was stirred at room temperature for 1 minute to produce a vinorelbine solution (containing 0.25 mg/mL vinorelbine). Furthermore, 0.05 mL of a vinorelbine preparation was extracted, and thereafter, 0.1 mL of the Polysorbate 80 solution produced in Example X-4 and 0.85 mL of the medium were then added to the vinorelbine preparation. The obtained mixture was stirred at room temperature for 1 minute to produce a vinorelbine solution (containing 0.5 mg/mL vinorelbine).

[Example X-6] Production of vinorelbine solution by addition of Polysorbate 80 in amount of about 1 part by mass with respect to 1 part by mass of vinorelbine

0.01 mL of a vinorelbine preparation was extracted, and thereafter, 0.01 mL of the Polysorbate 80 solution produced in Example X-4 and 0.97 mL of the medium were then added to the vinorelbine preparation. The obtained mixture was stirred at room temperature for 1 minute to produce a vinorelbine solution (containing 0.1 mg/mL vinorelbine). Moreover, 0.025 mL of a vinorelbine preparation was extracted, and thereafter, 0.025 mL of the Polysorbate 80 solution produced in Example X-4 and 0.95 mL of the medium were then added to the vinorelbine preparation. The obtained mixture was stirred at room temperature for 1 minute to produce a vinorelbine solution (containing 0.25 mg/mL vinorelbine). Furthermore, 0.05 mL of a vinorelbine preparation was extracted, and thereafter, 0.05 mL of the Polysorbate 80 solution produced in Example X-4 and 0.9 mL of the medium were then added to the vinorelbine preparation. The obtained mixture was stirred at room temperature for 1 minute to produce a vinorelbine solution (containing 0.5 mg/mL vinorelbine).

[Comparative Example X] Production of medium solution of vinorelbine

0.07 mL of a vinorelbine preparation was extracted, and 1.33 mL of the medium was then added to the vinorelbine preparation, followed by stirring the obtained mixture at room temperature for 1 minute, so as to produce a medium solution of vinorelbine (containing 0.5 mg/mL vinorelbine). Moreover, 0.6 mL of a solution was extracted from the produced 0.5 mg/mL vinorelbine solution, and 0.6 mL of the medium was then added thereto, followed by stirring the obtained mixture at room temperature for 1 minute, so as to produce a 0.25 mg/mL vinorelbine solution. Furthermore, 0.32 mL of a solution was extracted from the produced 0.25 mg/mL vinorelbine solution, and 0.48 mL of the medium was then added thereto, followed by stirring the obtained mixture at room temperature for 1 minute, so as to produce a 0.1 mg/mL vinorelbine solution.

Hereinafter, the present invention will be more specifically described in the following examples regarding oxaliplatin. However, these examples are not intended to limit the scope of the present invention. It is to be noted that the oxaliplatin preparation used in the following examples and the following comparative example is Oxaliplatin Intravenous Drip Infusion 50 mg "NK" (product name), which is a preparation containing an appropriate amount of phosphoric acid as an additive. The concentration of oxaliplatin in this preparation is 5 mg/mL.

[Example Y-1] Production of oxaliplatin solution by addition of Cremophor in amount of about 10 parts by mass with respect to 1 part by mass of oxaliplatin

0.152 mL (about 160 mg) of Cremophor (Kolliphor ELP, manufactured by Nippon Oil & Fats Co., Ltd.) was dissolved in 3.848 mL of the medium to produce a medium solution of Cremophor. The concentration of Cremophor in the solution was about 40 mg/mL. 0.1 mL of an oxaliplatin preparation was extracted, and thereafter, 0.125 mL of the produced Cremophor solution and 0.275 mL of the medium were then added to the oxaliplatin preparation. The obtained mixture was stirred at room temperature for 1 minute to produce an oxaliplatin solution (containing 1 mg/mL oxaliplatin). Moreover, 0.2 mL of an oxaliplatin preparation was extracted, and thereafter, 0.25 mL of the produced Cremophor solution and 0.05 mL of the medium were then added to the oxaliplatin preparation. The obtained mixture was stirred at room temperature for 1 minute to produce an oxaliplatin solution (containing 2 mg/mL oxaliplatin).

[Example Y-2] Production of oxaliplatin solution by addition of Cremophor in amount of about 5 parts by mass with respect to 1 part by mass of oxaliplatin

0.1 mL of an oxaliplatin preparation was extracted, and thereafter, 0.0625 mL of the Cremophor solution produced in Example Y-1 and 0.3375 mL of the medium were then added to the oxaliplatin preparation. The obtained mixture was stirred at room temperature for 1 minute to produce an oxaliplatin solution (containing 1 mg/mL oxaliplatin). Moreover, 0.2 mL of an oxaliplatin preparation was extracted, and thereafter, 0.125 mL of the Cremophor solution produced in Example Y-1 and 0.175 mL of the medium were then added to the oxaliplatin preparation. The obtained mixture was stirred at room temperature for 1 minute to produce an oxaliplatin solution (containing 2 mg/mL oxaliplatin).

[Example Y-3] Production of oxaliplatin solution by addition of Cremophor in amount of about 2 parts by mass with respect to 1 part by mass of oxaliplatin

0.076 mL (about 79.8 mg) of Cremophor (Kolliphor ELP, manufactured by Nippon Oil & Fats Co., Ltd.) was dissolved in 3.924 mL of the medium to produce a medium solution of Cremophor. The concentration of Cremophor in the solution was about 20 mg/mL. 0.16 mL of an oxaliplatin preparation was extracted, and thereafter, 0.08 mL of the Cremophor solution produced in Example Y-1 and 0.56 mL of the medium were then added to the oxaliplatin preparation. The obtained mixture was stirred at room temperature for 1 minute to produce an oxaliplatin solution (containing 1 mg/mL oxaliplatin). Moreover, 0.32 mL of an oxaliplatin preparation was extracted, and thereafter, 0.16 mL of the Cremophor solution produced in Example Y-1 and 0.32 mL of the medium were then added to the oxaliplatin preparation. The obtained mixture was stirred at room temperature for 1 minute to produce an oxaliplatin solution (containing 2 mg/mL oxaliplatin).

[Example Y-4] Production of oxaliplatin solution by addition of Cremophor in amount of about 1 part by mass with respect to 1 part by mass of oxaliplatin

0.16 mL of an oxaliplatin preparation was extracted, and thereafter, 0.04 mL of the Cremophor solution produced in Example Y-3 and 0.6 mL of the medium were then added to the oxaliplatin preparation. The obtained mixture was stirred at room temperature for 1 minute to produce an oxaliplatin solution (containing 1 mg/mL oxaliplatin). Moreover, 0.32 mL of an oxaliplatin preparation was extracted, and thereafter, 0.08 mL of the Cremophor solution produced in Example Y-1 and 0.4 mL of the medium were then added to the oxaliplatin preparation. The obtained mixture was stirred at room temperature for 1 minute to produce an oxaliplatin solution (containing 2 mg/mL oxaliplatin).

[Example Y-5] Production of oxaliplatin solution by addition of Polysorbate 80 in amount of about 2 parts by mass with respect to 1 part by mass of oxaliplatin

0.038 mL (about 40.4 mg) of Polysorbate 80 (manufactured by KANTO CHEMICAL CO., INC.) was dissolved in 3.962 mL of the medium to produce a medium solution of Polysorbate 80. The concentration of Polysorbate 80 in the solution was about 10 mg/mL. 0.16 mL of an oxaliplatin preparation was extracted, and thereafter, 0.16 mL of the produced Polysorbate 80 solution and 0.48 mL of the medium were then added to the oxaliplatin preparation. The obtained mixture was stirred at room temperature for 1 minute to produce an oxaliplatin solution (containing 1 mg/mL oxaliplatin). Moreover, 0.32 mL of an oxaliplatin preparation was extracted, and thereafter, 0.32 mL of the produced Polysorbate 80 solution and 0.16 mL of the medium were then added to the oxaliplatin preparation. The obtained mixture was stirred at room temperature for 1 minute to produce an oxaliplatin solution (containing 2 mg/mL oxaliplatin).

[Example Y-6] Production of oxaliplatin solution by addition of Polysorbate 80 in amount of about 1 part by mass with respect to 1 part by mass of oxaliplatin

0.16 mL of an oxaliplatin preparation was extracted, and thereafter, 0.08 mL of the Polysorbate 80 solution produced in Example Y-5 and 0.56 mL of the medium were then added to the oxaliplatin preparation. The obtained mixture was stirred at room temperature for 1 minute to produce an oxaliplatin solution (containing 1 mg/mL oxaliplatin). Moreover, 0.32 mL of an oxaliplatin preparation was extracted, and thereafter, 0.16 mL of the Polysorbate 80 solution produced in Example Y-5 and 0.32 mL of the medium were then added to the oxaliplatin preparation. The obtained mixture was stirred at room temperature for 1 minute to produce an oxaliplatin solution (containing 2 mg/mL oxaliplatin).

[Comparative Example Y] Production of medium solution of oxaliplatin

0.3 mL of an oxaliplatin preparation was extracted, and thereafter, 0.45 mL of the medium was then added to the oxaliplatin preparation. The obtained mixture was stirred at room temperature for 1 minute to produce a medium solution of oxaliplatin (containing 2 mg/mL oxaliplatin). Moreover, 0.25 mL of a solution was extracted from the produced 2 mg/mL oxaliplatin solution, and 0.25 mL of the medium was then added thereto, followed by stirring at room temperature for 1 minute, to produce a 1 mg/mL oxaliplatin solution.

[Test Example 1] Cytotoxicity Test of Fosaprepitant

< Experimental Method >

As cells, Balb/3T3 clone A31 cells (hereinafter abbreviated as "3T3 cells") obtained from JCRB Cell Bank were used. A normal saline (manufactured by Otsuka Pharmaceutical Co., Ltd.) was used as a negative control substance, and a normal saline aqueous solution of 0.1 mg/mL sodium laurate (manufactured by Nacalai Tesque, Inc.) (hereinafter abbreviated as "SDS") was used as a positive control substance. An aqueous solution of 1% Triton-X (manufactured by Sigma-Aldrich) was used for the measurement of the total activity of lactate dehydrogenase (hereinafter abbreviated as "LDH") present in the cells. Besides, for dilution of an analyte, a normal saline was used.

The cells were seeded at a cell density of 2 x 10000 cells/0.1 mL on a 96-well plate (manufactured by Corning International), and were then cultured in an incubator (at 37°C in 5% carbon dioxide) for 24 hours. Thereafter, the culture supernatant was removed from each well, and a sample (fosaprepitant with a concentration of 0.33 to 2.25 mg/mL) was exposed and was left at rest for 5 minutes. Three wells were used for each concentration. The SDS, the normal saline, and the 1% Triton-X aqueous solution were also exposed by the same operations as those for the sample.

With regard to the amount of LDH leaked into the supernatant, a total amount of culture supernatant recovered from each analyte was measured according to a JSCC transferable method using Hitachi 7180 Biochemistry Automatic Analyzer (manufactured by Hitachi High-Technologies Corporation). Using a mean value of the three wells for each concentration, a cytotoxicity percentage was calculated according to the following equation.

[Equation 1]

$$\text{Cytotoxicity percentage (\%)} = (\text{Amount of LDH in test substance}) / (\text{Amount of LDH in 1\% Triton-X}) \times 100$$

< Results >

The test results are shown in the following Table 1. The normal saline aqueous solution containing a fosaprepitant preparation, which was used as Comparative Example 1, contained 0.5 parts by mass of Polysorbate 80 with respect to 1 part by mass of fosaprepitant. As a result of the treatment of the cells with the 1.0 mg/mL drug, which was within the clinically administrable concentration range disclosed in Non-Patent Document 1 (since the drug (1 vial/150 mg drug) was used after it was diluted with 100 mL to 250 mL of a normal saline, the drug concentration became 1.5 mg/mL to 0.6 mg/mL), cytotoxicity was confirmed. When the drug was used at a drug concentration of 1.5 mg/mL, the expression of cytotoxicity was significant. In contrast, in the Examples of the present invention, the nonionic surfactant was comprised in an amount of 1 part by mass or more with respect to 1 part by mass of fosaprepitant, and a result, the improvement of cytotoxicity was confirmed compared with Comparative Example 1, and further, when the nonionic surfactant was comprised in an amount of 2 parts by mass or more, clear cytotoxicity was not exhibited, even though the drug was used at a drug concentration of 2.25 mg/mL. In Comparative Example 2 wherein neutralization was performed with hydrochloric acid up to pH 7.8, or in Comparative Example 3 wherein Macrogol 300 was added, the effect of suppressing cytotoxicity was not found, and further, the anionic surfactant SDS (positive control substance) exhibited cytotoxicity by itself. Accordingly, it was demonstrated that the phenomenon found in the present invention was the effects obtained by the nonionic surfactant.

[Table 1]

| Analyte | Fosaprepitant concentration (mg/mL) | Cytotoxicity percentage (%) |
|---|---|---|
| Negative control substance (normal saline) | - | 3 |
| Positive control substance (0.1 mg/mL SDS) | - | 32 |
| Comparative Example 1 (normal saline aqueous solution) * Containing 0.5 parts by mass of Polysorbate 80 as nonionic surfactant | 0.33 | 4 |
| | a | is |
| | 1.5 | 61 |
| | 2.25 | 92 |
| Comparative Example 2 Hydrochloric acid water added to Comparative Example 1 (pH 7.8) | 0.33 | 3 |
| | 1 | 29 |
| | 1.5 | 74 |
| | 2.25 | 94 |
| Comparative Example 3 6.7 Parts by mass of Macrogol 300 added to Comparative Example 1 | 0.33 | 4 |
| | 1 | 22 |
| | 1.5 | 64 |
| | 2.25 | 92 |

(continued)

| Analyte | Fosaprepitant concentration (mg/mL) | Cytotoxicity percentage (%) |
|---|---|---|
| Example 1 | 0.33 | 5 |
| | 1 | 5 |
| Containing about 7.2 parts by mass of nonionic surfactant | 1.5 | 7 |
| | 2.25 | 7 |
| Example 2 | 0.33 | 5 |
| | 1 | 9 |
| Containing about 3.8 parts by mass of nonionic surfactant | 1.5 | 11 |
| | 2.25 | 11 |
| Example 3 | 0.33 | 3 |
| | 1 | 8 |
| Containing about 30 parts by mass of nonionic surfactant | 1.5 | 9 |
| | 2.25 | 12 |
| Example 4 | 0.33 | 3 |
| | 1 | 7 |
| Containing about 2.0 parts by mass of nonionic surfactant | 1.5 | 10 |
| | 2.25 | 29 |
| Example 5 | 0.33 | 4 |
| | 1 | 10 |
| Containing about 1.0 part by mass of nonionic surfactant | 1.5 | 32 |
| | 2.25 | 62 |
| Example 6 | 0.33 | 3 |
| | 1 | 5 |
| Containing about 3.2 parts by mass of nonionic surfactant | 1.5 | 7 |
| | 2.25 | 10 |
| Example 7 | 0.33 | 5 |
| | 1 | 9 |
| Containing about 2.0 parts by mass of nonionic surfactant | 1.5 | 10 |
| | 2.25 | 14 |
| Example 8 | 0.33 | I |
| | 1 | 10 |
| Containing about 10 part by mass of nonionic surfactant | 7. 5 | 21 |
| | 2.25 | 49 |
| Example 9 | 0.33 | I |
| | 1 | 7 |
| Containing about 3.8 parts by mass of nonionic surfactant | 1.5 | 10 |
| | 2.25 | 11 |

[Test Example 2] Cytotoxicity Test of Vinorelbine

< Experimental Method >

As cells, 3T3 cells obtained from JCRB Cell Bank were used. A medium was used as a negative control. An aqueous solution of 1% Triton-X was used for the measurement of the total activity of LDH present in the cells.
The cells were seeded at a cell density of 2 x 10000 cells/0.1 mL on a 96-well plate, and were then cultured in an incubator (at 37°C in 5% carbon dioxide) for 24 hours. Thereafter, the culture supernatant was removed from each well, and a sample (vinorelbine with a concentration of 0.1 to 0.5 mg/mL) was exposed and was left at rest in the incubator for 15 minutes. The negative control was also exposed in the same manner as described above, and the 1% Triton-X solution was also exposed by the same operations as those described above for 1 minute. Three wells were used for each concentration, and after completion of the exposure, the supernatant was recovered.
The amount of LDH in each supernatant recovered was measured according to a JSCC transferable method using Hitachi 7180 Biochemistry Automatic Analyzer (manufactured by Hitachi High-Technologies Corporation). Using a mean value of the three wells for each concentration, a cytotoxicity percentage was calculated according to the following equation.

[Equation 2]

$$\text{Cytotoxicity percentage (\%)} = (\text{LDH in test substance}) - (\text{LDH in negative control}) / (\text{LDH in 1\% Triton-X}) \times 100$$

< Results >

The test results are shown in the following Table 2. With regard to the vinorelbine solution used as Comparative Example X, when the cells were treated with the 0.25 mg/mL drug, the vinorelbine solution was confirmed to have cytotoxicity. When the drug was used at a drug concentration of 0.5 mg/mL, the expression of cytotoxicity was significant. In contrast, in the Examples of the present invention, the nonionic surfactant was comprised in an amount of 1 part by mass or more with respect to 1 part by mass of vinorelbine, and a result, the improvement of cytotoxicity was confirmed compared with Comparative Example X. When Cremophor was comprised in an amount of 2 parts by mass or more, the cytotoxicity caused by the 0.25 mg/mL drug was almost completely suppressed. When Cremophor was comprised in an amount of 10 parts by mass, the cytotoxicity caused by the 0.5 mg/mL drug was almost completely suppressed. Since the cytotoxicity of the drug was suppressed in a nonionic surfactant content-dependent manner, it was demonstrated that the phenomenon found in the present invention was the effects obtained by the nonionic surfactant.

[Table 2]

| Analyte | | Vinorelbine concentration (mg/mL) | Cytotoxicity percentage (%) |
|---|---|---|---|
| Comparative Example X (vinorelbine solution) | | 0.1 | 1 |
| | | 0.25 | 11 |
| | | 0. 5 | 34 |
| Example X-1 | | 0.1 | 1 |
| | 10 Times weight of Cremophor added to | 0.25 | 0 |
| | Comparative Example X | 0.5 | 1 |
| Example X-2 | | 0.1 | 0 |
| | 2 Times weight of Cremophor added to Comparative | 0.25 | 2 |
| | Example X | 0.5 | 1 4 |
| Example X-3 | | 0.1 | 1 |

(continued)

| Analyte | | Vinorelbine concentration (mg/mL) | Cytotoxicity percentage (%) |
|---|---|---|---|
| | 1 Time weight of Cremophor added to Comparative | 0.25 | 3 |
| | Example X | 0.5 | 2 6 |
| Example X-4 | | 0.1 | 1 |
| | 10 Times weight of Polysorbate 80 added to | 0.25 | 2 |
| | Comparative Example X | 0.5 | 7 |
| Example X-5 | | 0.1 | 1 |
| | 2 Times weight of Polysorbate 80 added to | 0.25 | 3 |
| | Comparative Example X | 0.5 | 7 |
| Example X-6 | | 0.1 | 0 |
| | 1 Time weight of Polysorbate 80 added to | 0.25 | 6 |
| | Comparative Example X | 0.5 | 10 |

[Test Example 3] Cytotoxicity Test of Oxaliplatin

< Experimental Method >

As cells, 3T3 cells obtained from JCRB Cell Bank were used. A medium was used as a negative control. An aqueous solution of 1% Triton-X was used for the measurement of the total activity of LDH present in the cells.

The cells were seeded at a cell density of 2 x 10000 cells/0.1 mL on a 96-well plate and were then cultured in an incubator (at 37°C in 5% carbon dioxide) for 24 hours. Thereafter, the culture supernatant was removed from each well, and a sample (oxaliplatin with a concentration of 1 to 2 mg/mL) was exposed and was left at rest in the incubator for 2 hours. The negative control was also exposed in the same manner as described above. After completion of the exposure, the supernatant was removed from each well, and 100 μL of the 1% Triton-X aqueous solution was then added thereto to dissolve the cells remaining in each well. The obtained cell lysate was recovered. Three wells were used for each concentration.

The amount of LDH in each solution recovered was measured according to a JSCC transferable method using Hitachi 7180 Biochemistry Automatic Analyzer (manufactured by Hitachi High-Technologies Corporation). Using a mean value of the three wells for each concentration, a cytotoxicity percentage was calculated according to the following equation.

[Equation 3]

Cytotoxicity percentage (%) = 100 - (LDH remaining after test substance exposure) / (LDH remaining after negative control exposure) x 100

< Results >

The test results are shown in the following Table 3. With regard to the oxaliplatin solution used as Comparative Example Y, when the cells were treated with the 1 mg/mL drug, the oxaliplatin solution was confirmed to have cytotoxicity. When the drug was used at a drug concentration of 2 mg/mL, the expression of cytotoxicity was significant. In contrast, in the Examples of the present invention, Polysorbate 80 was comprised in an amount of 1 part by mass or more with respect to 1 part by mass of oxaliplatin, or Cremophor was comprised in an amount of 2 parts by mass or more with respect to 1 part by mass of oxaliplatin, and a result, the improvement of cytotoxicity was confirmed compared with Comparative Example Y Since the cytotoxicity of the drug was suppressed in a nonionic surfactant content-dependent manner, it was demonstrated that the phenomenon found in the present invention was the effects obtained by the nonionic surfactant.

[Table 3]

| Analyte | Oxaliplatin concentration (mg/mL) | Cytotoxicity percentage (%) |
|---|---|---|
| Comparative Example Y (oxaliplatin solution) | 1 | 32 |
| | 2 | 63 |
| Example Y-1 | 1 | 29 |
| 10 Times of added toweight Cremophor Comparative Example Y | 2 | 47 |
| Example Y-2 | 1 | 33 |
| 5 Times weight of Cremophor added to Comparative Example Y | 2 | 52 |
| Example Y-3 | 1 | 33 |
| 2 Times of added to weight Cremophor Comparative Example Y | 2 | 62 |
| Example Y-4 | 1 | 30 |
| 1 Time of added to weight Cremophor Comparative Example Y | 2 | 62 |
| Example Y-5 | 1 | 25 |
| 2 Times of 80 added toweight Polysorbate Comparative Example | 2 | 58 |
| YExample Y-6 | 1 | 29 |
| 1 Time weight of Polysorbate 80 added to Comparative Example Y | 2 | 51 |

[Test Example 4] Blood Vessel Stimulation Test of Fosaprepitant, Using Rabbit Posterior Auricular Vein

< Experimental Method >

The present test was carried out with reference to Non-Patent Document 6. Specifically, an indwelling needle was inserted into the posterior auricular vein of each male Japanese white rabbit (purchased from Oriental Yeast Co., Ltd.), and thereafter, using a syringe pump (TERUMO CORPORATION), a sample prepared by diluting Example 1, 4, 6 or 7 or Comparative Example 1 with a normal saline to a fosaprepitant concentration of 1.5 mg/mL was continuously administered into the vein at an administration rate of 0.4 mL/min for 60 minutes. A normal saline (manufactured by Otsuka Pharmaceutical Co., Ltd.) was used as a negative control, and was administered in the same manner as that described above. Two rabbits were used in each group. About twenty-four hours after the administration, the animals were euthanized, and the auricle was collected from each animal and was then fixed with a 10% neutral buffered formalin solution (manufactured by FUJIFILM Wako Pure Chemical Corporation).

A total of 4 tissue sections containing blood vessels were cut out of the formalin-fixed auricle, specifically from the sections every 5 mm from the site 20 mm closer to the heart than the blood vessel insertion site of the indwelling needle. A hematoxylin and eosin-stained specimen was prepared from each tissue section, and a histopathological test was then carried out.

In the histopathological test, with regard to blood vessel specimens collected from 4 sites in each animal, 5 findings, namely, changes in blood vessels (disappearance of vascular endothelial cells and thrombosis) and changes around the blood vessels (bleeding, cell infiltration, and edema) were each evaluated with 5 grades (0: no change; 1+: mild degree; 2+: moderate degree; 3+: strong degree; and 4+: severe degree). The thus evaluated grades 0 to 4+ were each digitized to points 0 to 4, and the obtained points were defined to be pathological scores. For each of the 4 sites of vascular tissues, the scores of the total findings of each group were summed up, and the obtained scores were then divided by the number of animals, so as to calculate the group average score of each site. Further, the scores of the 4 sites were summed up, and the obtained scores were then divided by the number of animals, so as to obtain the average score of each group.

Non-Patent Document 6: Blood Vessel Stimulation Test of Antitumor Drug Paclitaxel in Rabbits, The Journal of Toxicological Sciences, Volume 19, pp. 123-130, 1994

< Results >

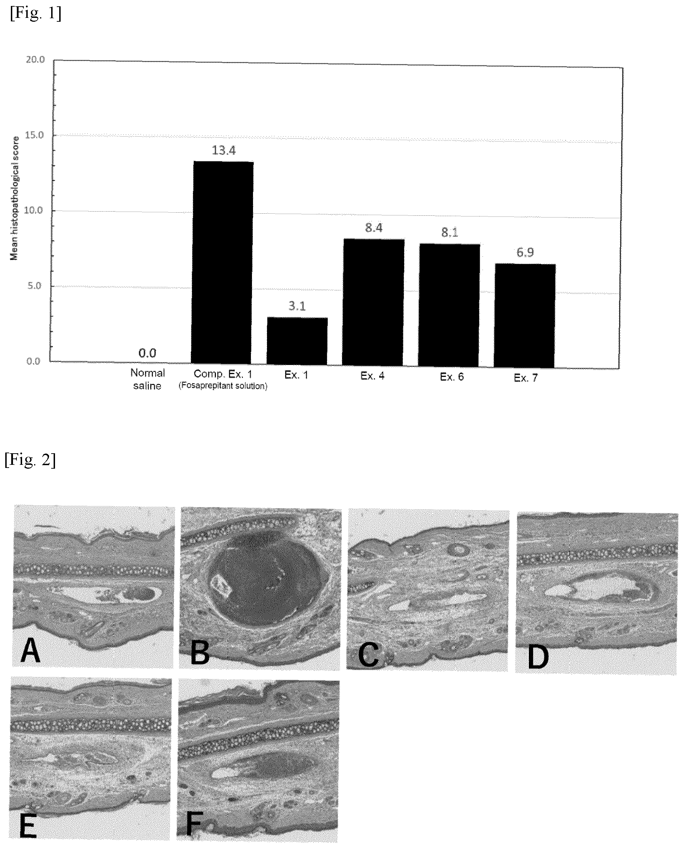

The results of the histopathological test regarding blood vessel stimulation, wherein the rabbit posterior auricular vein was used, are shown in Table 4, Fig. 1, and Fig. 2. Two rabbits were used in each group, and the blood vessels in 4 sites of the posterior auricular vein were evaluated for each animal. Table 4 shows the strongest changes, which were observed. (Grades - 0: no change; 1+: mild degree; 2+: moderate degree; 3+: strong degree; and 4+: severe degree). The fosaprepitant solution used as Comparative Example 1 exhibited a clear vascular stimulation image and pathological scores, compared with the normal saline as a negative control. Thus, it was considered that the present stimulation would be caused by fosaprepitant. On the other hand, in the Examples of the present invention, the nonionic surfactant was comprised in an amount of 2 parts by mass or more with respect to 1 part by mass of fosaprepitant, and thus, vascular stimulation was attenuated compared with the fosaprepitant solution of Comparative Example 1. Moreover, when Cremophor was comprised in an amount of about 6.7 parts by mass (about 7.2 parts by mass of the nonionic surfactant; Example 1) with respect to 1 part by mass of fosaprepitant, significant effects were obtained. From these phenomena, it was demonstrated that the nonionic surfactant of the present invention has the effect of suppressing vascular stimulation.

[Table 4]

| Test substance | Findings | | | | |
|---|---|---|---|---|---|
| | Disappearance of vein endothelial cells | Thrombosis | Bleeding | Inflammatory cell infiltration | Edema around vein |
| Negative control Normal saline | 0 | 0 | 0 | 0 | 0 |
| Comparative Example 1 Fosaprepitant solution | 3 + | 3 + | 2 + | 3 + | 3 + |
| Example 1 Containing about 7.2 parts by mass of nonionic surfactant | 2 + | 1 + | 1 + | 1 + | 2 + |
| Example 4 Containing about 2.0 parts by mass of nonionic surfactant | 3 + | 3 + | 1 + | 2 + | 2 + |
| Example 6 Containing about 3.2 parts by mass of nonionic surfactant | 3 + | 3 + | 1 + | 2 + | 2 + |
| Example 7 Containing about 2.0 parts by mass of nonionic surfactant | 3 + | 2 + | 1 + | 1 + | 2 + |

Claims

1. A composition containing a water-soluble drug causing vascular disorder, wherein the composition comprises 1 part by mass or more of a nonionic surfactant with respect to 1 part by mass of the water-soluble drug causing vascular disorder.

2. The composition containing a water-soluble drug causing vascular disorder according to claim 1, wherein the composition comprises 2 parts by mass or more of the nonionic surfactant with respect to 1 part by mass of the water-soluble drug causing vascular disorder.

3. The composition containing a water-soluble drug causing vascular disorder according to claim 1 or 2, wherein the nonionic surfactant is one or more selected from the group consisting of polyoxyethylene castor oil (Cremophor), polysorbate, polyoxyethylene hydrogenated castor oil, and polyoxyethylene polyoxypropylene glycol.

4. The composition containing a water-soluble drug causing vascular disorder according to any one of claims 1 to 3, which is a preparation containing a water-soluble drug causing vascular disorder.

5. The composition containing a water-soluble drug causing vascular disorder according to any one of claims 1 to 3, which is an administration solution containing a water-soluble drug causing vascular disorder.

6. A solution comprising a nonionic surfactant, for use in preparation of an administration solution containing a water-soluble drug causing vascular disorder, wherein the administration solution prepared using the solution containing a water-soluble drug causing vascular disorder comprising the nonionic surfactant comprises 1 part by mass or more of the nonionic surfactant with respect to 1 part by mass of the water-soluble drug causing vascular disorder.

7. The solution comprising a nonionic surfactant, for use in preparation of an administration solution containing a water-soluble drug causing vascular disorder according to claim 6, which is used to prepare an administration solution comprising a water-soluble drug causing vascular disorder, containing 2 parts by mass or more of the nonionic surfactant with respect to 1 part by mass of the water-soluble drug causing vascular disorder.

8. The solution comprising a nonionic surfactant for use in preparation of an administration solution containing a water-soluble drug causing vascular disorder according to claim 6 or 7, wherein the nonionic surfactant is one or more selected from the group consisting of polyoxyethylene castor oil (Cremophor), polysorbate, polyoxyethylene hydrogenated castor oil, and polyoxyethylene polyoxypropylene glycol.

9. A kit including a set of a water-soluble drug preparation causing vascular disorder, and a solution comprising a nonionic surfactant for use in preparation of an administration solution containing a water-soluble drug causing vascular disorder, wherein the administration solution containing a water-soluble drug causing vascular disorder that is prepared by dissolving the water-soluble drug preparation causing vascular disorder in the solution comprises 1 part by mass or more of the nonionic surfactant with respect to 1 part by mass of the water-soluble drug causing vascular disorder.

10. The kit according to claim 9, wherein the administration solution containing a water-soluble drug causing vascular disorder that is prepared by dissolving the water-soluble drug preparation causing vascular disorder in the dissolving solution becomes an administration solution containing a water-soluble drug causing vascular disorder, comprising 2 parts by mass or more of the nonionic surfactant with respect to 1 part by mass of the water-soluble drug causing vascular disorder.

11. The kit according to claim 9 or 10, wherein the nonionic surfactant is one or more selected from the group consisting of polyoxyethylene castor oil (Cremophor), polysorbate, polyoxyethylene hydrogenated castor oil, and polyoxyethylene polyoxypropylene glycol.

12. An agent for suppressing vascular disorder for a water-soluble drug causing vascular disorder, wherein the agent for suppressing vascular disorder comprises a nonionic surfactant, wherein 1 part by mass or more of the nonionic surfactant is used with respect to 1 part by mass of the water-soluble drug causing vascular disorder.

13. The agent for suppressing vascular disorder for a water-soluble drug causing vascular disorder, according to claim 12, wherein 2 parts by mass or more of the nonionic surfactant is used with respect to 1 part by mass of the water-soluble drug causing vascular disorder.

14. The agent for suppressing vascular disorder for a water-soluble drug causing vascular disorder, according to claim 12 or 13, wherein the nonionic surfactant is one or more selected from the group consisting of polyoxyethylene castor oil (Cremophor), polysorbate, polyoxyethylene hydrogenated castor oil, and polyoxyethylene polyoxypropylene glycol.

**15.** A solution containing a nonionic surfactant, which is used in combination with a water-soluble drug causing vascular disorder, wherein 1 part by mass or more of the nonionic surfactant is used with respect to 1 part by mass of the water-soluble drug causing vascular disorder.

**16.** The solution according to claim 15, wherein 2 parts by mass or more of the nonionic surfactant is used with respect to 1 part by mass of the water-soluble drug causing vascular disorder.

**17.** The solution according to claim 15 or 16, wherein the nonionic surfactant is one or more selected from the group consisting of polyoxyethylene castor oil (Cremophor), polysorbate, polyoxyethylene hydrogenated castor oil, and polyoxyethylene polyoxypropylene glycol.

**18.** The composition containing a water-soluble drug causing vascular disorder, the solution for use in preparation of an administration solution containing a water-soluble drug causing vascular disorder, the kit, the agent for suppressing vascular disorder, or the solution according to any one of claims 1 to 17, wherein the water-soluble drug causing vascular disorder is a drug selected from the group consisting of fosaprepitant, a vinca alkaloid-based drug, and a platinum-based drug.

**19.** The composition containing a water-soluble drug causing vascular disorder, the solution for use in preparation of an administration solution containing a water-soluble drug causing vascular disorder, the kit, the agent for suppressing vascular disorder, or the solution according to any one of claims 1 to 17, wherein the water-soluble drug causing vascular disorder is vinorelbine.

**20.** The composition containing a water-soluble drug causing vascular disorder, the solution for use in preparation of an administration solution containing a water-soluble drug causing vascular disorder, the kit, the agent for suppressing vascular disorder, or the solution according to any one of claims 1 to 17, wherein the water-soluble drug causing vascular disorder is oxaliplatin.

[Fig. 1]

[Fig. 2]

# INTERNATIONAL SEARCH REPORT

| International application No. |
| --- |
| **PCT/JP2021/042404** |

| A. | CLASSIFICATION OF SUBJECT MATTER |
| --- | --- |

*A61K 31/675*(2006.01)i; *A61K 9/08*(2006.01)i; *A61K 47/10*(2006.01)i; *A61K 47/26*(2006.01)i; *A61K 47/44*(2017.01)i; *A61P 9/00*(2006.01)i
FI:   A61K31/675; A61K9/08; A61K47/10; A61K47/26; A61K47/44; A61P9/00

According to International Patent Classification (IPC) or to both national classification and IPC

| B. | FIELDS SEARCHED |
| --- | --- |

Minimum documentation searched (classification system followed by classification symbols)

A61K31/675; A61K9/08; A61K47/10; A61K47/26; A61K47/44; A61P9/00

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

JSTPlus/JMEDPlus/JST7580 (JDreamIII); CAplus/REGISTRY/MEDLINE/EMBASE/BIOSIS (STN)

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| X | CN 1771954 A (TANG, Xing) 17 May 2006 (2006-05-17)<br>    claims 1-2, page 8, lines 11-15, page 12, lines 3-4 | 1-19 |
| X | JP 2008-505972 A (SD PHARMACEUTICALS, INC.) 28 February 2008 (2008-02-28)<br>    claims 1-3, 6, paragraphs [0010]-[0013], [0059] | 1-19 |
| Y | | 18, 20 |
| X | CN 1868455 A (SHENYANG PHARMACEUTICAL UNIV.) 29 November 2006<br>(2006-11-29)<br>    claim 1, page 4, line 14, page 5, lines 19-30, page 6, lines 28-32 | 1-18, 20 |
| Y | | 18, 20 |
| Y | 小野薬品工業株式会社, [ブロイメンド点滴静注用150mg], 添付文書, April 2020,<br>pages 1-6, (ONO PHARMACEUTICAL CO., LTD.), non-official translation (Proimend for<br>intravenous drip infusion 150mg. attached document.)<br>    particularly, [11. 2 その他の副作用], [19. 有効成分に関する理化学的知見], non-<br>official translation (11. 2 other side effects, 19. physicochemical knowledge about active<br>ingredients.) | 18, 20 |

| ✓ | Further documents are listed in the continuation of Box C. | ✓ | See patent family annex. |
| --- | --- | --- | --- |

| * | Special categories of cited documents: |
| --- | --- |
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
| --- | --- |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
| --- | --- |
| **04 January 2022** | **18 January 2022** |

| Name and mailing address of the ISA/JP | Authorized officer |
| --- | --- |
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

## INTERNATIONAL SEARCH REPORT

International application No.

**PCT/JP2021/042404**

**C.    DOCUMENTS CONSIDERED TO BE RELEVANT**

| Category* | Citation of document, with indication, where appropriate, of the relevant passages | Relevant to claim No. |
|---|---|---|
| A | RUBA, B. et al. Surfactant effects on lipid-based vesicles properties: a review. Journal of Pharmaceutical Sciences. 2018, vol. 107, issue 5, pages 1237-1246<br>table 3 | 1-20 |

Form PCT/ISA/210 (second sheet) (January 2015)

**INTERNATIONAL SEARCH REPORT**
Information on patent family members

International application No.

**PCT/JP2021/042404**

| Patent document cited in search report | | | Publication date (day/month/year) | Patent family member(s) | Publication date (day/month/year) |
|---|---|---|---|---|---|
| CN | 1771954 | A | 17 May 2006 | (Family: none) | |
| JP | 2008-505972 | A | 28 February 2008 | US 2006/0008480 A1 claims 1-3, 6, paragraphs [0012]-[0015], [0059] | |
| CN | 1868455 | A | 29 November 2006 | (Family: none) | |

Form PCT/ISA/210 (patent family annex) (January 2015)

## REFERENCES CITED IN THE DESCRIPTION

**Patent documents cited in the description**

- JP 3073770 B

- JP 2019073457 A

**Non-patent literature cited in the description**

- Measures for Alleviating Phlebitis Caused by Fosaprepitant Injection. *Japanese Journal of Cancer and Chemotherapy,* 2015, vol. 42 (3), 323-326
- Book for Fully Understanding about Cancer Therapeutic Drugs. Shorinsha Inc,
- *Journal of Japanese Society of Pharmaceutical Oncology,* April 2020, vol. 14
- Blood Vessel Stimulation Test of Antitumor Drug Paclitaxel in Rabbits. *The Journal of Toxicological Sciences,* 1994, vol. 19, 123-130